# EUROPÄISCHE PATENTSCHRIFT

(11) **EP 2 516 360 B1**
(45) Veröffentlichungstag und Bekanntmachung des Hinweises auf die Patenterteilung: **23.07.2014**
(21) Anmeldenummer: 10796381.1
(22) Anmeldetag: 20.12.2010
(51) Int. Cl.: C07C 5/25, C07C 11/02

(54) **ISOMERISIERUNG VON LINEAREN ALPHA-OLEFINEN**
ISOMERIZING LINEAR ALPHA OLEFINS
ISOMÉRISATION D'ALPHA-OLÉFINES LINÉAIRES

(30) Priorität: 22.12.2009 EP 09180313
(43) Veröffentlichungstag der Anmeldung: 31.10.2012
(73) Patentinhaber: BASF SE, 67056 Ludwigshafen (DE)
(72) Erfinder: KÖNIGSMANN, Lucia, 68159 Mannheim (DE); SCHWAB, Ekkehard, 67434 Neustadt (DE); HAHN, Thilo, 67292 Kirchheimbolanden (DE); KONS, Germain, Iselin New Jersey 07302 (US)
(86) Internationale Anmeldenummer: PCT/EP2010/070207
(87) Internationale Veröffentlichungsnummer: WO 2011/076718

(56) Entgegenhaltungen:
- EP-A2- 0 841 090
- WO-A1-2008/124375
- US-A- 3 919 340

## Beschreibung

Die Erfindung betrifft ein Verfahren zur Isomerisierung von linearen alpha-Olefinen.

Lineare alpha-Olefine werden aus Ethylen hergestellt. Anschließend wird die terminale (= alpha) C=C-Doppelbindung nach "innen" zu den thermodynamisch begünstigen linearen internen Olefinen umgelagert. Derartige isomerisierte Olefine werden als lineare interne Olefine (LIO) bezeichnet und sind wertvolle Produkte zur Herstellung von ASA (alkenyl succinic anhydride). ASA wird in der Papierindustrie eingesetzt. Die Herstellung von ASA erfolgt durch Reaktion der LIO mit MSA (Maleinsäureanhydrid).

Zur Doppelbindungsisomerisierung von Olefinen sind zahlreiche Verfahren bekannt, die in einem weiten Temperaturbereich von unter 100 °C bis über 250 °C durchgeführt werden. Das Temperaturniveau hat dabei einen entscheidenden Einfluss auf die Isomerenzusammensetzung. Derartige Isomerisierungsreaktionen können sowohl mit als auch ohne Zusatz von Wasserstoff durchgeführt werden. Jedoch können als Nebenreaktionen in Abwesenheit von Wasserstoff Oligomerisierung und Skelletisomerisierung auftreten. Die Isomerisierung in Anwesenheit von Wasserstoff kann die Doppelbindung hydrieren und zu gesättigten Produkte führen. Um die Isomerisierung bei minimaler Hydrierung der Doppelbindung wirtschaftlich durchzuführen ist bekannt, ein Gasgemisch aus Wasserstoff und Stickstoff einzusetzen, wobei der Zusatz an Wasserstoff niedrig gehalten wird.

Aus der EP 0 841 090 A2 ist ein Festbettkatalysator und ein kontinuierliches Verfahren zur Herstellung von 2-Buten-1-ol-Verbindungen bekannt, wobei der Festbettkatalysator Palladium und Selen oder Tellur oder ein Gemisch aus Selen und Tellur auf einem Siliciumdioxidträger enthält. Aus der WO 2008/124375 A1 ist ein Verfahren zur Herstellung interner Olefine durch katalytische Isomerisierung von alpha-Olefinen bekannt, bei welchem als Katalysator Kaliumcarbonat oder Kaliumacetat auf einem Oxidträger verwendet werden. Aus der WO 2009/050194 A1 ist bekannt, Olefine mit 4 bis 20 C-Atomen, insbesondere Buten, in Gegenwart eines heterogenen Katalysators zu isomerisieren, der auf einem Aluminiumoxidträger Nickel und mindestens ein Element der Gruppe VIB enthält. Aus der US-A-4 417 089 ist die Hydroisomerisierung von terminalen Olefinen an einem Katalysator bekannt, der im Wesentlichen aus einer PalladiumKomponente, einer Cer-Komponente und Aluminium besteht.

Aus der EP 0 636 676 A1 und der korrespondierenden US-A-5,569,806 sowie der EP 0 636 677 A1 und der korrespondierenden US-A-5,502,269 ist bereits bekannt, die Isomerisierung von alpha-Olefinen zu inneren Olefinen mit einem Palladium basierten Katalysator durchzuführen, der mit einer Schwefel-Komponente beladen ist.

Das Dokument US 3 919 340 beschreibt isomerisierbare Kohlenwasserstoffe unter Verwendung einer katalytischen Zusammensetzung, umfassend eine Kombination aus einer Platin- oder Palladium-Komponente, einer Iridium-Komponente, einer Germanium-Komponente und einer Halogenkomponente mit einem porösen Trägermaterial.

Nachteilig an den bekannten Verfahren sind zu geringe Ausbeuten, beispielsweise durch Nebenreaktionen, wie Verzweigung, geringe Selektivität und hohe Preise der Katalysatoren.

Der Erfindung lag die Aufgabe zugrunde, ein verbessertes Verfahren zur Isomerisierung, insbesondere Hydroisomerisierung, von linearen alpha-Olefinen mit 10 bis 25 C-Atomen zur Verfügung zu stellen, welches eine verbesserte Ausbeute und Selektivität ermöglicht.

Die Erfindung betrifft ein Verfahren zur Isomerisierung von linearen alpha-Olefinen mit 10 bis 25 C-Atomen zu linearen internen Olefinen an einem heterogenen Katalysator, dadurch gekennzeichnet, dass der heterogene Katalysator Palladium und Selen und/oder Tellur auf einem Träger enthält.

Der Katalysator weist also als wesentliche Bestandteile Palladium und Selen oder Palladium und Tellur oder Palladium und Selen zuzüglich Tellur auf.

Bevorzugt ist ein Katalysator, der Palladium und Selen oder Tellur oder ein Gemisch aus Selen und Tellur auf einem Siliciumdioxidträger enthält und eine BET-Oberfläche von 80 bis 380 m²/g und ein Porenvolumen von 0,6 bis 0,95 cm³/g im Porendurchmesserbereich von 3 nm bis 300 µm aufweist, wobei 80 bis 95% des Porenvolumens im Porendurchmesserbereich von 10 bis 100 nm liegen.

Vorzugsweise enthält der Katalysator 0,1 bis 2,0 Gew.-% Palladium und 0,01 bis 0,2 Gew.-% Selen, Tellur oder eines Gemisches aus Selen und Tellur, bezogen auf das Gesamtgewicht des Katalysators. In einer bevorzugten Ausführungsform weist der Katalysator eine Selen-Dotierung in einer Menge von 0,01 bis 0,07 Gew.-% bezogen auf das Gesamtgewicht des Katalysators auf.

Vorzugsweise beträgt die BET-Oberfläche 100 bis 150 m²/g, insbesondere 110 bis 130 m²/g. Die BET-Oberfläche wird dabei bestimmt durch N₂-Adsorption gemäß DIN 66131.

Insbesondere enthält der Katalysator 0,2 bis 0,8 Gew.-%, insbesondere 0,4 bis 0,6 Gew.-% Palladium. Vorzugsweise enthält der Katalysator 0,02 bis 0,08, insbeson dere 0,04 bis 0,06 Gew.-% Selen, Tellur oder eines Gemisches aus Selen und Tellur, vorzugsweise Selen.

Neben den genannten aktiven Komponenten können weitere Metalle in geringem Umfang auf dem Katalysator vorliegen. Vorzugsweise liegen nur Palladium, Selen und/oder Tellur, insbesondere nur Palladium und Selen auf dem Siliciumdioxidträger vor.

Die erfindungsgemäß zu verwendenden Katalysatoren können nach beliebigen geeigneten Verfahren hergestellt werden. Vorzugsweise werden sie hergestellt durch Tränken eines Siliciumdioxidträgers mit einer Lösung einer Palladiumverbindung und einer Selenverbindung oder Tellurverbindung oder eines Gemisches einer Selenverbindung und Tellurverbindung. Dabei können eine oder mehrere Palladiumverbindungen, Selenverbindungen und/oder Tellurverbindungen eingesetzt werden. Vorzugsweise werden die Verbindungen in Form von wässrigen Lösungen eingesetzt. Dabei wird Palladium vorzugsweise in Form von Salzen, wie Palladiumnitrat, oder Komplexen eingesetzt. Selen und/oder Tellur werden beispielsweise in oxidischer Form eingesetzt. Weitere geeignete Palladium-, Selen- und Tellurverbindungen sind beschrieben in der DE-A-27 51 766. Dabei kann der Siliciumdioxidträger nacheinander mit Lösungen der einzelnen Verbindungen in beliebiger Reihenfolge getränkt werden, wobei zwischen den einzelnen Tränkungsschritten der Katalysatorträger getrocknet werden kann. Der Katalysatorträger kann jedoch auch mit einer Lösung getränkt werden, die die Verbindungen der aktiven Substanzen in einem entsprechenden gewünschten Verhältnis enthält. Die Konzentration der Lösungen kann so gewählt werden, dass die gewünschte Menge an Palladium und Selen und/oder Tellur durch einmaliges Tränken auf den Katalysator aufgebracht werden kann. Ein Aufbringen durch mehrmaliges Tränken ist jedoch auch möglich.

Vorzugsweise wird der Katalysatorträger in der Lösung der aktiven Substanzen bewegt, sodann der getränkte Katalysator bei einer Temperatur von etwa 120 °C getrocknet und anschließend bei einer Temperatur von etwa 200 °C getempert. Vor oder bei dem Einsatz des Katalysators in der Isomerisierung werden die aktiven Substanzen, d. h. Palladium und Selen und/oder Tellur in Gegenwart von Wasserstoff reduziert.

Der Katalysatorträger wird vorzugsweise hergestellt, indem Siliciumdioxid aus einer Alkalisilikatlösung ausgefällt und getrocknet und zu Formkörpern gepresst wird, und die so hergestellten Formkörper bei einer Temperatur im Bereich von 400 bis 1100 °C, vorzugsweise 600 bis 900 °C, insbesondere 800 bis 900 °C kalziniert werden.

Dabei wird beispielsweise wässrige ammoniakalische Alkalisilikatlösung vorgelegt und mit wässriger Schwefelsäure behandelt, so dass Siliciumdioxid ausfällt. Der erhaltene Niederschlag kann sodann abfiltriert, gewaschen und sprühgetrocknet werden. Dabei wird die Sprühtrocknung vorzugsweise so durchgeführt, dass das gewonnene Siliciumdioxidpulver einen Wassergehalt besitzt, der einem Glühverlust von 25 bis 35 Gew.-% beim Glühen bei 900 °C für 2 Stunden entspricht. Das erhaltene Siliciumdioxidpulver kann sodann mit einem Peptisierungsmittel angeteigt und in die gewünschte Form gebracht werden. Bei der Verwendung als Festbettkatalysator kann dieser alle geeigneten makroskopischen Formen aufweisen, beispielsweise in Form von Strängen, Tabletten, beliebig geformten Pellets, Kugeln oder Ringen eingesetzt werden. Vorzugsweise wird das Siliciumdioxidpulver zu Strängen verpresst. Die Stränge werden sodann bei 120 bis 150 °C getrocknet und anschließend bei 400 bis 1100 °C, vorzugsweise 600 bis 900 °C, insbesondere 800 bis 900 °C kalziniert.

Andere Herstellungsverfahren für den Siliciumdioxidträger können gewählt werden, sofern die erhaltenen Träger die angegebene BET-Oberfläche, Porengröße und Porengrößenverteilung aufweisen.

In einer besonders bevorzugten Ausführungsform wird der Katalysator als Festbettkatalysator eingesetzt.

### Reaktor

Die erfindungsgemäße Isomerisierung kann in jeder beliebigen Vorrichtung durchgeführt werden, in der eine kontinuierliche Verfahrensführung möglich ist. Vorzugsweise wird die Isomerisierung in Rieselfahrweise in einem Rohrreaktor durchgeführt, der den erfindungsgemäß zu verwendenden Festbettkatalysator enthält. Der Rohrreaktor enthält dabei vorzugsweise im oberen Teil eine Gasverteilung, beispielsweise in Form einer Filterplatte, eines statischen Mischers oder einer Düse. Die Gasverteilung dient zum Zuführen von Gasgemisch (Wasserstoff/Stickstoff), wobei der Reaktorquerschnitt vorzugsweise gleichmäßig begast wird. Die zu isomerisierende Verbindung wird dabei zunächst über eine Heizzone geleitet, mit dem Gas vermischt und in den Reaktor geleitet. Die Katalysatorbelastung wird dabei so eingestellt, dass am Reaktorausgang ein Umsatz von vorzugsweise 70 bis 99 %, besonders bevorzugt 90 bis 99 % erzielt wird. Die Wasserstoffbegasung wird in Abhängigkeit von Temperatur und Gesamtdruck so eingestellt, dass ein Wasserstoffpartialdruck von 0,1 bis 5 bar, vorzugsweise 0,1 bis 2 bar, insbesondere 0,1 bis 1 bar aufrechterhalten wird. Der durchgeleitete Wasserstoff kann im Reaktoraustrag nach Auskondensation von Leichtsiedern als Abgas gefahren werden oder wieder in den Prozess zurückgeführt werden.

### Verfahrensparameter

Die Isomerisierung wird vorzugsweise bei einem Druck von 0,5 bis 5 bar absolut, insbesondere 0,8 bis 2 bar absolut, durchgeführt.

Die Isomerisierung wird dabei bei Temperaturen zwischen 80 und 150 °C, vorzugsweise 120 °C bis 140 °C durchgeführt. In einer anderen bevorzugten Ausführungsform wird die Isomerisierung bei 100 bis 120 °C durchgeführt. Dabei werden je nach eingesetzter Ausgangsverbindung Katalysatorbelastungen von 0,5 bis 5 I/I(Katalysator) x h, vorzugsweise 1 bis 3 I/I(Katalysator) x h gefahren.

In einer bevorzugten Ausführungsform wird die Isomerisierung in Gegenwart von Wasserstoff oder eines Gemisches aus Wasserstoff und Inertgas, vorzugsweise Stickstoff, durchgeführt. In einer ganz besonders bevorzugten Ausführungsform ist das bei der Isomerisierung eingesetzte Inertgas Stickstoff, wobei der eingesetzte Wasserstoff in einer Menge von 5 bis 50 mol% bezogen auf Stickstoff eingesetzt wird.

Das erfindungsgemäße Verfahren kann in Anwesenheit oder in Abwesenheit eines inerten organischen Lösungsmittels durchgeführt werden. Verwendbare inerte organische Lösungsmittel sind beispielsweise Ether, wie Diethylether, Dioxan, Tetrahydrofuran, Alkohole, wie Ethanol, Isobutanol, aromatische oder aliphatische Kohlenwasserstoffe, wie Heptan oder Benzol oder Gemische davon. Vorzugsweise wird ohne inertes organisches Lösungsmittel gearbeitet.

### Ausgangsstoffe

Die zu isomerisierenden linearen alpha-Olefine können als einheitliche Verbindungen oder als Mischung aus Kohlenwasserstoff mit unterschiedlichen Kettenlängen vorliegen. Beispiele kommerziell erhältlicher linearer alpha-Olefine sind C16-C18-Olefine. In einer bevorzugten Ausführungsform ist das lineare alpha-Olefin ein Monoolefin. Besonders bevorzugt ist das lineare alpha-Olefin mit der Summenformel C₁₈H₃₆.

Bei der erfindungsgemäßen Isomerisierung verschiebt sich die Doppelbindung vorzugsweise in die 2-Position. Bevorzugte Produkte bei der Isomerisierung von 1-Oktadecen sind neben 2-Oktadecen 3-Oktadecen und 4-Oktadecen.

Die erfindungsgemäß isomerisierten Olefine eignen sich insbesondere zu Herstellung von ASA (alkenyl succinic anhydride).

In einer bevorzugten Ausführungsform wird das erfindungsgemäße Verfahren zur Isomerisierung der Olefine mit 10 bis 25 C-Atomen in Abwesenheit von niedrigeren Homologen, insbesondere in Abwesenheit von Butenen, durchgeführt.

### Beispiele

### Katalysatorherstellung

In einem Rührbehälter wurde eine wässrige ammoniakalische Natriumsilikatlösung vorgelegt. Mit wässriger Schwefelsäure wurde unter Rühren Siliciumdioxid ausgefällt. Der erhaltene Niederschlag wurde abfiltriert, gewaschen und anschließend sprühgetrocknet. Die Sprühtrocknung wurde dabei so durchgeführt, dass das gewonnene Siliciumdioxidpulver einen Wassergehalt besitzt, der einem Glühverlust zwischen 25 und 35 Gew.-% in 2 Stunden bei 900 °C entspricht. Das so erhaltene Siliciumdioxidpulver wurde mit Wasser und Ammoniak als Peptisierungsmittel angeteigt und zu Strängen mit 3 mm Durchmesser verpresst. Die Stränge wurden in einem Trockenschrank bei 120 bis 150 °C getrocknet und anschließend bei 820 bis 870 °C kalziniert.

300 g des so gewonnenen Trägermaterials in Form von Strängen mit 3 mm Durchmesser wurden in einem Rundkolben an einem Rotationsverdampfer mit einer wässrigen Lösung aus 13,64 g Palladiumnitratlösung mit 11 Gew.-% Palladium und 0,21 g SeO₂ in 244 g destilliertem Wasser versetzt. Der Kolben wurde durch Rotation bei Zimmertemperatur so lange bewegt, bis die gesamte Lösung vom Trägermaterial aufgenommen wurde. Anschließend wurde der Kolben mit dem Katalysator unter Rotation auf 120 °C erhitzt und bei einer Drehgeschwindigkeit von 9 Umdrehungen pro Minute unter Einleitung von 2000 I Luft pro Stunde in 3 Stunden getrocknet. Nach dem Trocknen wurde die Temperatur unter ständiger Rotation des Kolbens und Einleitung von 1000 I Luft/h auf 200 °C erhöht und der Katalysator für 3 Stunden getempert.

Der so erhaltene Siliciumdioxid Trägerkatalysator enthielt 0,5 Gew.-% Palladium und 0,05 Gew.-% Selen, bezogen auf das Gesamtgewicht des Katalysators. Die BET-Oberfläche beträgt 119 m²/g und das Porenvolumen 0,82 cm³/g im Porendurchmesserbereich von 3 nm bis 300 µm. Von diesem Porenvolumen lagen 91,7 % im Porendurchmesserbereich von 10 nm bis 100 nm.

### Reaktor

Als Reaktor wurde ein Doppelmantelglasreaktor mit einem Innendurchmesser von 6 mm und einer Länge von 124 cm verwendet. Er wurde mit dem oben angegebenen Katalysator gefüllt und mit 5 l/h Stickstoff gespült. Anschließend wurde der Katalysator eine Stunde bei 150 °C und 20 l/h Wasserstoff durch Reduktion aktiviert.

### Beispiele

Der mit dem erfindungsgemäß zu verwendenden Katalysator gefüllter Reaktor wurde unter folgenden Bedingungen gestartet:
**Beispiel 1**
   Zulauf von
   20 g/h 1-Oktadecen
   2,5 l/h Wasserstoff (H₂) und
   3,5 l/h Stickstoff (N₂)
   Temperatur: 140 °C
   Laufzeit: 6 Tage
   Katalysatorbelastung: 2,2 g / ml x h
   Über die gesamte Laufzeit wurde bei Normaldruck in der Sumpffahrweise gefahren. Dabei wurde >98% Umsatz und 90% Selektivität erreicht.
**Beispiel 2**
   Zulauf von
   20 g/h 1-Oktadecen
   2,5 l/h Wasserstoff (H₂) und
   3,5 l/h Stickstoff (N₂)
   Temperatur: 140 °C
   Laufzeit: 5 Tage
   Katalysatorbelastung: 2,2 g / ml x h
   Über die gesamte Laufzeit wurde bei Normaldruck in der Rieselfahrweise gefahren. Dabei wurde 93% Umsatz und 89% Selektivität erreicht.
**Beispiel 3**
   Zulauf von
   50 g/h 1-Oktadecen
   0,1-0,3 l/h Wasserstoff (H₂) und
   2,9 l/h Stickstoff (N₂)
   Temperatur: 115-140 °C
   Laufzeit: 14 Tage
   Über die gesamte Laufzeit wurde bei Normaldruck im Rieselfahrweise gefahren bei einer Katalysator Belastung von 3,6g /ml x h. Dabei wurde 70 % Umsatz erreicht bei
   >99 % Selektivität.

Es wurde folgende Produktverteilung erhalten:
Tabelle 1:

| | **Produktverteilung** | | | | | | |
|---|---|---|---|---|---|---|---|
| **Beispiele** | **1-Oktadecene Gew%** | **2-Oktadecene Gew%** | **3-Oktadecene Gew%** | **4+-Oktadecene Gew%** | **Oktadecane Gew%** | **Umsatz %** | **Selektivität zu LIO %** |
| Beispiel 1 | 1,4 | 37,1 | 19,4 | 32,4 | 9,7 | 98,6 | 89,9 |
| Beispiel 2 | 6,9 | 44,8 | 15,3 | 23,1 | 9,9 | 93,08 | 89,4 |
| Beispiel 3 | 29,8 | 52,5 | 7,9 | 9,6 | 0,2 | 69,5 | 99,7 |

Aus den oben angegebenen Werten ergibt sich, dass der erfindungsgemäß zu verwendende Katalysator in Verbindung mit der gewählten Fahrweise insbesondere hinsichtlich der Selektivität zu den gewünschten LIO's ein sehr gutes Eigenschaftsprofil aufweist.

## Patentansprüche

1. Verfahren zur Isomerisierung von linearen alpha-Olefinen mit 10 bis 25 C-Atomen zu linearen internen Olefinen an einem heterogenen Katalysator, **dadurch gekennzeichnet, dass** der Katalysator Palladium und Selen und/oder Tellur auf einem Träger enthält.

2. Verfahren nach Anspruch 1, **dadurch gekennzeichnet, dass** der Träger im Wesentiichen aus Siliciumdioxid besteht.

3. Verfahren nach wenigstens einem der vorhergehenden Ansprüche, **dadurch gekennzeichnet, dass** ein Katalysator verwendet wird, der Palladium und Selen oder Tellur oder Palladium und ein Gemisch aus Selen und Tellur auf einem Siliciumdioxidträger enthält, eine BET-Oberfläche von 80 bis 380 m²/g, ein Porenvolumen von 0,6 bis 0,95 cm³/g und einen Porendurchmesser von 3 nm bis 300 µm aufweist, wobei 80 bis 95 % des Porenvolumens im Porendurchmesserbereich von 10 bis 100 nm liegen.

4. Verfahren nach wenigstens einem der vorhergehenden Ansprüche, **dadurch gekennzeichnet, dass** der Katalysator 0,1 bis 2,0 Gew.-% Palladium und 0,01 bis 0,2 Gew.-% Selen, Tellur oder ein Gemisch aus Selen und Tellur, bezogen auf das Gesamtgewicht des Katalysators, enthält.

5. Verfahren nach wenigstens einem der vorhergehenden Ansprüche, **dadurch gekennzeichnet, dass** der Katalysator eine BET-Oberfläche von 100 bis 150 m²/g aufweist.

6. Verfahren nach wenigstens einem der vorhergehenden Ansprüche, **dadurch gekennzeichnet, dass** der Katalysator eine Dotierung von Se in einer Menge von 0,01-0,07 % aufweist.

7. Verfahren nach wenigstens einem der vorhergehenden Ansprüche, **dadurch gekennzeichnet, dass** das alpha-Olefin ein Olefin mit 16 bis 18 C-Atomen ist.

8. Verfahren nach wenigstens einem der vorhergehenden Ansprüche, **dadurch gekennzeichnet, dass** das lineare alpha-Olefin ein Monoolefin ist.

9. Verfahren nach wenigstens einem der vorhergehenden Ansprüche, **dadurch gekennzeichnet, dass** die Isomerisierung bei einer Temperatur von 100 bis 120 °C durchgeführt wird.

10. Verfahren nach wenigstens einem der vorhergehenden Ansprüche, **dadurch gekennzeichnet, dass** die Isomerisierung bei einem Druck von 0,5 bis 5 bar absolut durchgeführt wird.

11. Verfahren nach wenigstens einem der vorhergehenden Ansprüche, **dadurch gekennzeichnet, dass** die Isomerisierung in Gegenwart von Wasserstoff oder eines Gemisches aus Wasserstoff und Inertgas, vorzugsweise Stickstoff, durchgeführt wird.

12. Verfahren nach Anspruch 11, **dadurch gekennzeichnet, dass** das bei der Isomerisierung eingesetzte Inertgas Stickstoff (N₂) ist und der eingesetzte Wasserstoff vorzugsweise in einer Menge von 5 bis 50 mol% bezogen auf N₂ eingesetzt wird.

13. Verfahren nach wenigstens einem der vorhergehenden Ansprüche, **dadurch gekennzeichnet, dass** die Isomerisierung an einem Festbettkatalysator durchgeführt wird.

## Claims

1. A process for isomerizing linear alpha-olefins having from 10 to 25 carbon atoms to form linear internal olefins over a heterogeneous catalyst, wherein the catalyst comprises palladium and selenium and/or tellurium on a support.

2. The process according to claim 1, wherein the support comprises essentially silicon dioxide.

3. The process according to at least one of the preceding claims, wherein a catalyst which comprises palladium and selenium or tellurium or palladium and0 a mixture of selenium and tellurium on a silicon dioxide support and has a BET surface area of from 80 to 380 m²/g, a pore volume of from 0.6 to 0.95 cm³/g and a pore diameter of from 3 nm to 300 µm, with from 80 to 95% of the pore volume being in the pore diameter range from 10 to 100 nm, is used.

4. The process according to at least one of the preceding claims, wherein the catalyst comprises from 0.1 to 2.0% by weight of palladium and from 0.01 to 0.2% by weight of selenium, tellurium or a mixture of selenium and tellurium, based on the total weight of the catalyst.

5. The process according to at least one of the preceding claims, wherein the catalyst has a BET surface area of from 100 to 150 m²/g.

6. The process according to at least one of the preceding claims, wherein the catalyst is doped with 0.01-0.07% of Se.

7. The process according to at least one of the preceding claims, wherein the alpha-olefin is an olefin having from 16 to 18 carbon atoms.

8. The process according to at least one of the preceding claims, wherein the linear alpha-olefin is a monoolefin.

9. The process according to at least one of the preceding claims, wherein the isomerization is carried out at a temperature of from 100 to 120°C.

10. The process according to at least one of the preceding claims, wherein the isomerization is carried out at a pressure of from 0.5 to 5 bar absolute.

11. The process according to at least one of the preceding claims, wherein the isomerization is carried out in the presence of hydrogen or a mixture of hydrogen and inert gas, preferably nitrogen.

12. The process according to claim 11, wherein the inert gas used in the isomerization is nitrogen (N₂) and the hydrogen used is preferably used in an amount of from 5 to 50 mol% based on N₂.

13. The process according to at least one of the preceding claims, wherein the isomerization is carried out over a fixed-bed catalyst.

## Revendications

1. Procédé d'isomérisation d'alpha-oléfines linéaires contenant 10 à 25 atomes C en oléfines internes linéaires sur un catalyseur hétérogène, **caractérisé en ce que** le catalyseur contient du palladium et du sélénium et/ou du tellure sur un support.

2. Procédé selon la revendication 1, **caractérisé en ce que** le support est essentiellement constitué de dioxyde de silicium.

3. Procédé selon au moins l'une quelconque des revendications précédentes, **caractérisé en ce qu'**un catalyseur qui contient du palladium et du sélénium ou du tellure ou du palladium et un mélange de sélénium et de tellure sur un support en dioxyde de silicium, qui présente une surface BET de 80 à 380 m²/g, un volume poreux de 0,6 à 0,95 cm³/g et un diamètre de pore de 3 nm à 300 µm est utilisé, 80 à 95 % du volume poreux se trouvant dans la plage de diamètres de pores allant de 10 à 100 nm.

4. Procédé selon au moins l'une quelconque des revendications précédentes, **caractérisé en ce que** le catalyseur contient 0,1 à 2,0 % en poids de palladium et 0,01 à 0,2 % en poids de sélénium, de tellure ou d'un mélange de sélénium et de tellure, par rapport au poids total du catalyseur.

5. Procédé selon au moins l'une quelconque des revendications précédentes, **caractérisé en ce que** le catalyseur présente une surface BET de 100 à 150 m²/g.

6. Procédé selon au moins l'une quelconque des revendications précédentes, **caractérisé en ce que** le catalyseur présente un dopage de Se en une quantité de 0,01 à 0,07 %.

7. Procédé selon au moins l'une quelconque des revendications précédentes, **caractérisé en ce que** l'alpha-oléfine est une oléfine contenant 16 à 18 atomes C.

8. Procédé selon au moins l'une quelconque des revendications précédentes, **caractérisé en ce que** l'alpha-oléfine linéaire est une monooléfine.

9. Procédé selon au moins l'une quelconque des revendications précédentes, **caractérisé en ce que** l'isomérisation est réalisée à une température de 100 à 120 °C.

10. Procédé selon au moins l'une quelconque des revendications précédentes, **caractérisé en ce que** l'isomérisation est réalisée à une pression de 0,5 à 5 bar absolu.

11. Procédé selon au moins l'une quelconque des revendications précédentes, **caractérisé en ce que** l'isomérisation est réalisée en présence d'hydrogène ou d'un mélange d'hydrogène et d'un gaz inerte, de préférence l'azote.

12. Procédé selon la revendication 11, **caractérisé en ce que** le gaz inerte utilisé lors de l'isomérisation est l'azote (N₂) et l'hydrogène utilisé est de préférence utilisé en une quantité de 5 à 50 % en moles par rapport à N₂.

13. Procédé selon au moins l'une quelconque des revendications précédentes, **caractérisé en ce que** l'isomérisation est réalisée sur un catalyseur en lit fixe.
